(19) Europäisches Patentamt
European Patent Office
Office européen des brevets

(11) **EP 4 356 919 A1**

(12) **EUROPEAN PATENT APPLICATION**
published in accordance with Art. 153(4) EPC

(43) Date of publication:
**24.04.2024  Bulletin 2024/17**

(21) Application number: **22861376.6**

(22) Date of filing: **24.08.2022**

(51) International Patent Classification (IPC):
*A61K 35/76* (2015.01)       *A61K 31/43* (2006.01)
*A61K 31/545* (2006.01)     *A61K 45/00* (2006.01)
*A61P 31/04* (2006.01)       *A61P 43/00* (2006.01)

(52) Cooperative Patent Classification (CPC):
**A61K 31/43; A61K 31/545; A61K 35/76;
A61K 45/00; A61P 31/04; A61P 43/00**

(86) International application number:
**PCT/JP2022/031783**

(87) International publication number:
**WO 2023/027088 (02.03.2023 Gazette 2023/09)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **25.08.2021   JP 2021137252**

(71) Applicants:
• **Incorporated Educational Institution Rakuno
  Gakuen
  Ebetsu-shi, Hokkaido, 069-8501 (JP)**
• **NDTS Co., Ltd.
  Sapporo-shi, Hokkaido 004-0015 (JP)**

(72) Inventors:
• **IWANO Hidetomo
  Ebetsu-shi Hokkaido 069-8501 (JP)**
• **FUJIKI Jumpei
  Ebetsu-shi Hokkaido 069-8501 (JP)**
• **HIGUCHI Hidetoshi
  Ebetsu-shi Hokkaido 069-8501 (JP)**
• **GONDAIRA Satoshi
  Ebetsu-shi Hokkaido 069-8501 (JP)**

• **USUI Masaru
  Ebetsu-shi Hokkaido 069-8501 (JP)**
• **MURATA Ryou
  Ebetsu-shi Hokkaido 069-8501 (JP)**
• **NAKAMURA Tomohiro
  Ebetsu-shi Hokkaido 069-8501 (JP)**
• **NISHIDA Keita
  Ebetsu-shi Hokkaido 069-8501 (JP)**
• **INOUE Hiroki
  Sapporo-shi Hokkaido 004-0015 (JP)**
• **TAMURA Yutaka
  Sapporo-shi Hokkaido 004-0015 (JP)**
• **IWANO Naomi
  Sapporo-shi Hokkaido 004-0015 (JP)**

(74) Representative: **Wasner, Marita
  Niizuma Wasner GmbH
  Patentanwaltskanzlei
  Postfach 278
  4125 Riehen (CH)**

Remarks:
The complete document including Reference
Table(s) and the Sequence Listing(s) can be
downloaded from the EPO website

(54) **COMPOSITION FOR IMPROVING DRUG SENSITIVITY OF ANTIBACTERIAL DRUG TO METHICILLIN-RESISTANT STAPHYLOCOCCUS AUREUS (MRSA), COMPOSITION FOR TREATING OR PREVENTING MRSA INFECTION, AND COMPOSITION FOR REDUCING VIRULENCE OF MRSA**

(57)   This composition for improving the drug sensitivity of an antibacterial drug to methicillin-resistant Staphylococcus aureus (MRSA) comprises at least one bacteriophage selected from the group consisting of the bacteriophage identified by accession number NITE BP-693 and the bacteriophage identified by accession number NITE BP-694.

**EP 4 356 919 A1**

**Description**

Technical Field

The present invention relates to a composition for increasing the drug sensitivity of methicillin-resistant Staphylococcus aureus (MRSA) to an antibacterial drug, a composition for treating or preventing an MRSA infection, and a composition for reducing the virulence of MRSA.

Background Art

With the recent rapid increase in the number of cases of infections with methicillin-resistant Staphylococcus aureus (MRSA) all over the world, not only in Japan, MRSA has been considered as a clinically problematic causal bacterium for serious infections, and examinations have been conducted on therapeutic agents therefor.

Bacteriophages, a collective term for viruses that infect bacteria, infect bacteria as a host and then grow in the bacteria. Daughter phages resulting from the growth are discharged out of the bacterial cells through the cell walls of the bacteria, and the bacteria undergo bacteriolysis to die. In addition, bacteriophages are advantageous, for example, in that they do not influence on other bacterial florae than pathogenic bacteria that serve as their hosts, and that they are easy to grow and large amounts of bacteriophages can be prepared at low cost.

In view of the advantages, attempts have been made to use a bacteriophage as a bacteriocide or the like for bacteria, and several research results on disinfection against Staphylococcus aureus (S. aureus) with use of a bacteriophage have been reported.

In Patent Literature 1, the present inventors have found and reported bacteriophages having a characteristic to cause bacteriolysis for S. aureus, which are a bacteriophage identified by accession number NITE BP-693 and that identified by accession number NITE BP-694.

Moreover, Non Patent Literature 1 suggests the possibility that applying the phage PYO$^{Sa}$ followed by an antibacterial drug is more effective against S. aureus than applying an antibacterial drug alone.

Citation List

Patent Literature

Patent Literature 1: Japanese Patent Laid-Open No. 2011-50373

Non Patent Literature

Non Patent Literature 1: Proc Natl Acad Sci U S A. 2021 Mar 9; 118 (10): e2008007118. doi:10.1073/pnas.2008007118. Brandon A Berryhill et al, Evaluating the potential efficacy and limitations of a phage for joint antibiotic and phage therapy of Staphylococcus aureus infections

Summary of Invention

Technical Problem

While attempts to search for therapeutic methods for MRSA infections have been made, the diversification of MRSA infections is under progress: for example, community-acquired MRSA infections is becoming more frequent; and livestock-associated MRSA has become an issue in the world. Under such a situation, therapeutic strategy suitable for individual cases of MRSA infections has been more strongly demanded, whereas new approaches against MRSA are still to be developed.

The present invention was made in view of such circumstances, and an object of the present invention is to provide a composition capable of increasing the drug sensitivity of MRSA to an antibacterial drug via a specific bacteriophage contained therein, a composition for treating or preventing an MRSA infection, and a composition for reducing the virulence of MRSA.

Solution to Problem

To achieve the object, a composition according to a first aspect of the present invention for increasing the drug sensitivity of methicillin-resistant Staphylococcus aureus (MRSA) to an antibacterial drug contains:
at least one bacteriophage selected from the group consisting of a bacteriophage identified by accession number NITE BP-693 and a bacteriophage identified by accession number NITE BP-694.

For example, the drug sensitivity is increased by allowing MRSA to acquire resistance to the bacteriophage.

For example, the antibacterial drug is a β-lactam antibacterial drug.

A composition according to a second aspect of the present invention for treating or preventing an MRSA infection contains:
at least one bacteriophage selected from the group consisting of a bacteriophage identified by accession number NITE BP-693 and a bacteriophage identified by accession number NITE BP-694; and an antibacterial drug.

For example, MRSA is allowed to acquire resistance to the bacteriophage.

For example, the antibacterial drug is a β-lactam antibacterial drug.

A composition according to a third aspect of the present invention for reducing the virulence of MRSA contains:
at least one bacteriophage selected from the group consisting of a bacteriophage identified by accession number NITE BP-693 and a bacteriophage identified by accession number NITE BP-694.

For examples the virulence is reduced by allowing MRSA to acquire resistance to the bacteriophage.

Advantageous Effects of Invention

The present invention can provide a composition capable of increasing the drug sensitivity of MRSA to an antibacterial drug via a specific bacteriophage contained therein, a composition for treating or preventing an MRSA infection, and a composition for reducing the virulence of MRSA.

Brief Description of Drawings

[Figure 1] Figure 1(a) is a graph for examining the infectivity of a phage for φSA012-resistant MRSA (mutants: SAm1-201, SAm1-106), and Figure 1(b) is photographs for the results.

[Figure 2] Figure 2 is a graph for examining the growth-inhibitory effect of a phage (φSA012) for an MRSA wild-type strain.

[Figure 3] Figure 3 is a graph for examining the growth-inhibitory effect of a phage (φSA012) for φSA012-resistant MRSA (mutant: SAm1-106).

[Figure 4] Figure 4 is a graph for examining the growth-inhibitory effect of a phage (φSA012) for φSA012-resistant MRSA (mutant: SAm1-201).

[Figure 5] Figure 5 is a graph for examining the growth-inhibitory effect of a phage and an antibacterial drug (oxacillin) both added from the beginning of test to a wild-type strain of MRSA.

[Figure 6] Figure 6 is a graph for examining differential expressions of genes associated with virulence in an MRSA mutant.

[Figure 7] Figure 7 is a graph for examining differential expressions of genes associated with drug efflux pumps in an MRSA mutant.

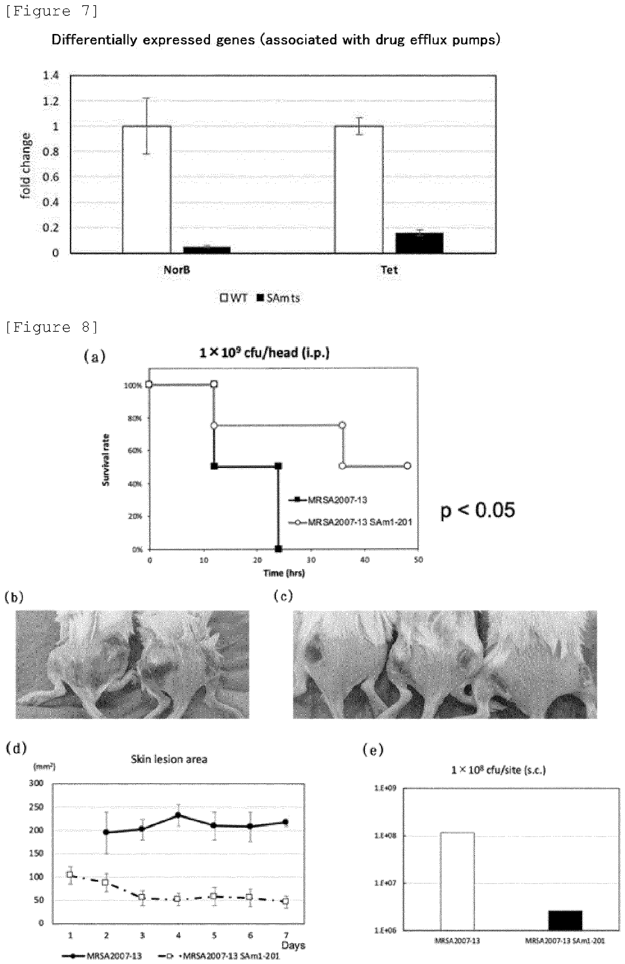

[Figure 8] Figure 8(a) is a graph showing the survival rates of female 8-week-old BALB/c mice after intraperitoneal administration of a wild-type strain (MRSA2007-13 strain) or a phage-induced mutant (phage-resistant bacterium MRSA2007-13 SAm1-201) thereto, Figure 8(b) is a photograph showing the gross appearance of lesion sites in female 8-week-old BALB/c mice 7 days after subcutaneous administration of a wild-type strain (MRSA2007-13 strain) thereto, Figure 8(c) is a photograph showing the gross appearance of lesion sites 7 days after subcutaneous administration of a phage-induced mutant (SAm1-201), Figure 8(d) is a graph showing transitions of abscess size (mean ± SE) over 7 days after administration, and Figure 8(e) is a graph for evaluating bacterial counts in tissues at lesion sites in mice 7 days after administration with use of LB agar (1 mouse per group (n = 1)).

Description of Embodiments

(1. Composition for increasing drug sensitivity of MRSA to antibacterial drug)

The composition of the present invention is a composition for increasing the drug sensitivity of methicillin-resistant Staphylococcus aureus (MRSA) to an antibacterial drug. The composition contains at least one bacteriophage selected from the group consisting of a bacteriophage identified by accession number NITE BP-693 and a bacteriophage identified by accession number NITE BP-694.

One of the bacteriophages to serve as an active ingredient of the composition of the present invention is a bacteriophage deposited as accession number NITE BP-693 in NITE Patent Microorganisms Depositary, Biological Resource Center, National Institute of Technology and Evaluation (2-5-8 Kazusakamatari, Kisarazu-shi, Chiba 2920818, Japan) on December 25, 2008. The present inventors have named the bacteriophage "φSA012".

The other of the bacteriophages to serve as an active ingredient of the composition of the present invention is a bacteriophage deposited as accession number NITE BP-694 in NITE Patent Microorganisms Depositary, Biological Resource Center, National Institute of Technology and Evaluation (2-5-8 Kazusakamatari, Kisarazu-shi, Chiba 2920818, Japan) on December 25, 2008. The present inventors have named the bacteriophage "φSA039".

Each of φSA012 and φSA039 has been understood to have almost the same size as a T4 phage and be slightly longer than it through observation with a transmission electron microscope. φSA012 and φSA039 each have a contractible sheath, hence being inferred to belong to the Myoviridae family. For other structures and characteristics of the bacteriophages according to the present invention, see descriptions in Japanese Patent Laid-Open No. 2011-50373.

The bacteriophages according to the present invention can be obtained by submitting a request for furnishing to the depositary in which those are deposited. Alternatively, the bacteriophages can be obtained from wastewater or the like collected from a domestic sewage flow or a sewage treatment facility, or from a biological sample, for example, from blood, a nasal cavity, a pharynx, skin, or an intestinal tract derived from an animal infected with S. aureus through separation/purification with a conventional method such as a plaque assay/separation method described in Japanese Patent Laid-Open No. 2011-50373. The bacteriophages according to the present invention can be grown with a common growth method for bacteriophages such as a plate lysate method described in Japanese Patent Laid-Open No. 2011-50373.

The composition of the present invention at least needs to contain any of the bacteriophages described above. Applicable are, for example, a bacteriophage solution obtained with the mentioned growth method, and a product obtained from the bacteriophage solution through separation from the culture residue by centrifugation or the like.

Details of the bacteriophage to be contained in the composition of the present invention are as described above. The bacteriophage to be contained in the composition of the present invention may be either one strain or a bacteriophage mix solution (phage cocktail) of φSA012 and φSA039. An additional bacteriophage may be further contained. Examples of the additional bacteriophage include, but are not limited to, a bacteriophage against a bacterium differing from MRSA according to the present invention (e.g., S. aureus), and the additional bacteriophage may be a temperate bacteriophage, without being limited to bacteriolytic one.

Those skilled in the art could appropriately adjust the concentration of the bacteriophage in the composition of the present invention in view of the form of usage without limitation; for example, the concentration in using as a liquid dosage form is preferably $1 \times 10^3$ to $1 \times 10^{13}$ PFU/mL (PFU: plaque-forming unit), more preferably $1 \times 10^5$ to $1 \times 10^{11}$ PFU/mL, and even more preferably $1 \times 10^8$ to $1 \times 10^{10}$ PFU/mL.

Herein, methods for determining whether the drug sensitivity to an antibacterial drug has increased are, for example, as follows: (i) a disk method (drug sensitivity test) is applied to determine the diameter of an inhibitory zone for the sensitivity of MRSA to an antibacterial drug and the diameter of an inhibitory zone for the sensitivity of MRSA to the same antibacterial drug with application of the bacteriophage according to the present invention, and if the latter diameter is larger than the former dimeter, the drug sensitivity to the antibacterial drug can be determined to have increased; and (ii) an MIC method (drug sensitivity test) is applied to determine an MIC (minimum inhibitory concentration) value for the sensitivity of MRSA to an antibacterial drug and an MIC value for the sensitivity of MRSA to the same antibacterial drug with application of the bacteriophage according to the present invention, and if the latter MIC value is smaller than the former MIC value, the drug sensitivity to the antibacterial drug can be determined to have increased.

For example, the drug sensitivity of MRSA to an antibacterial drug may be increased by allowing MRSA to acquire resistance to the bacteriophage according to the present invention. Herein, "acquire resistance" means that MRSA comes to have resistance to the bacteriophage according to the present invention to make the bacteriophage ineffective to MRSA (e.g., the growth of MRSA is prevented from being inhibited by the bacteriophage) or make the bacteriophage less effective to MRSA (e.g., the degree of growth inhibition for MRSA by the bacteriophage decreases). Determination of whether resistance to a bacteriophage has been acquired is, for example, as follows: the growth activity of MRSA with application of the bacteriophage according to the present invention is measured through EOP (plaque-forming rate) or turbidimetry described later, and whether "resistance has been acquired" can be determined by comparing the growth activity with that before application of the bacteriophage. Alternatively, for example, genetic analysis can determine that "resistance has been acquired" if it detects a mutation for mgrA, femA, or both of them.

Examples of the antibacterial drug include, but are not limited to, β-lactam antibacterial drugs, aminoglycoside antibacterial drugs, lincomycin antibacterial drugs, fosfomycin antibacterial drugs, tetracycline antibacterial drugs, chloramphenicol antibacterial drugs, macrolide antibacterial drugs, ketolide antibacterial drugs, polypeptide antibacterial drugs, glycopeptide antibacterial drugs, streptogramin antibacterial drugs, quinolone antibacterial drugs, and oxazolidinone.

The antibacterial drug is preferably a β-lactam antibacterial drug. Examples of the β-lactam antibacterial drug can include penicillin antibacterial drugs (penicillin G, ampicillin, bacampicillin, lenampicillin, cyclacillin, amoxicillin, pivmecillin, aspoxicillin, cloxacillin, piperacillin, methicillin); combination penicillin antibacterial drugs (ampicillin, cloxacillin); β-lactamase inhibitor-containing penicillin antibacterial drugs (ampicillin, sulbactam, clavulanic acid, amoxicillin, piperacillin, tazobactam); cephem antibacterial drugs (cefazolin, cephalothin, cephalexin, cefatrizine, cefroxadine, cefaclor, cefadroxil, cefotiam, cefmetazole, flomoxef, cefminox, cefbuperazone, cefuroxime, axetil, cefdinir, cefditoren, pivoxil, cefteram, cefpodoxime, proxetil, cefcapene, cefotaxime, ceftriaxone, cefoperazone, cefmenoxime, ceftazidime, ceftibuten, cefixime, cefodizime, latamoxef, ceftizoxime, cefpirome, cefozopran, cefepime); β-lactamase inhibitor-containing cephem antibacterial drugs (cefoperazone, sulbactam); carbapenem antibacterial drugs (imipenem, cilastatin, panipenem, betamipron, meropenem, biapenem, doripenem, tebipenem); monobactam antibacterial drugs (aztreonam, sulbactam, tazobactam, carumonam); and penem antibacterial drugs (faropenem).

For example, the composition of the present invention is administered to subjects affected by an MRSA infection, for example, mammals such as rodents including mice, rats, hamsters, and guinea pigs, primates including humans, chimpanzees, and rhesus monkeys, livestock including pigs, bovines, goats, horses, sheep, and avians, and pet animals including dogs and cats. Humans are preferred subjects.

For example, the composition of the present invention can be formulated into a dosage form such as a tablet, a granule, a powder, a capsule, a syrup, and an injection by means of a conventional method, and a proper drug delivery system (DDS) may be used. In addition, diluents, binders, lubricants, coloring agents, disintegrators, thickeners, preservatives, stabilizers, pH adjusters, and others that are used in common pharmaceuticals can be added. The dosage of the composition can be appropriately set according to the age, body weight, indication, and others of a subject individual.

(2. Composition for treating or preventing MRSA infection)

The composition provided by the present invention for treating or preventing an MRSA infection contains: at least one bacteriophage selected from the group consisting of a bacteriophage identified by accession number NITE BP-693 and a bacteriophage identified by accession number NITE BP-694; and an antibacterial drug.

The composition of the present invention elicits the effect of an antibacterial drug to treat or prevent an MRSA infection by increasing the drug sensitivity of MRSA to the antibacterial drug via the bacteriophage according to the present invention (as described above).

Herein, an "MRSA infection" refers to an infection due to MRSA such as respiratory infection, bacteremia, infective endocarditis, skin/soft tissue infection, intraabdominal infection, bone/joint infection, central nervous system infection, urinary tract infection, and pediatric infection. The composition of the present invention can be used for treating or preventing any of those MRSA infections, and for preventing postoperative infection.

Herein, "treating an MRSA infection" encompasses medically acceptable therapeutic interventions for various purposes, including delaying or stopping the progression of an MRSA infection, amelioration or temporary amelioration of an MRSA infection, disinfection, decolonization, inhibition of growth, and the like for MRSA, and prevention of the recurrence of an MRSA infection. Herein, "preventing an MRSA infection" encompasses medically acceptable preventive interventions for various purposes, including preventing the onset of or having an MRSA infection and preventing infection with MRSA.

For example, the effect of an antibacterial drug to prevent or treat an MRSA infection may be elicited by allowing MRSA to acquire resistance to a bacteriophage that serves as an active ingredient of the composition of the present invention, thereby increasing the drug sensitivity of MRSA to the antibacterial drug. How to determine whether "resistance has been acquired" is as described above.

The antibacterial drug is as described above, and may be, for example, a β-lactam antibacterial drug. Details of the β-lactam antibacterial drug are as described above.

The composition of the present invention is administered to subjects affected by an MRSA infection, for example, mammals such as rodents including mice, rats, hamsters, and guinea pigs, primates including humans, chimpanzees, and rhesus monkeys, livestock including pigs, bovines, goats, horses, sheep, and avians, and pet animals including dogs and cats. Humans are preferred subjects.

For example, the composition of the present invention can be formulated into a dosage form such as a tablet, a granule, a powder, a capsule, a syrup, and an injection by means of a conventional method, and a proper drug delivery system (DDS) may be used. In addition, diluents, binders, lubricants, coloring agents, disintegrators, thickeners, preservatives, stabilizers, pH adjusters, and others that are used in common pharmaceuticals can be added. The dosage of the composition can be appropriately set according to the age, body weight, indication, and others of a subject individual.

Examples of methods of administrating the composition of the present invention include (i) a method of using the bacteriophage according to the present invention and an antibacterial drug in combination (this method may be cocktail therapy, or use of a drug combination); and (ii) a method of administering the bacteriophage according to the present invention and then administering an antibacterial drug. Administration may be performed at any time, for example, during a meal, after a meal, before a meal, between meals, or before sleeping.

The composition of the present invention may further contain an additional drug, or be used in combination therewith. In using in combination, simultaneous administration or administration at different times may be applied. The "additional drug" is not limited as long as the additional drug does not cancel the effect of the bacteriophage according to the present invention to increase the drug sensitivity to an antibacterial drug, and a drug according to the mode of usage can be appropriately selected for use from those conventionally used for different purposes, such as antiinflammatory agents, immunosuppressive agents, prophylactic agents for infections, therapeutic agents for infections, insecticides, anthelmintic agents, antifungal agents, and antiviral agents.

(3. Composition for reducing virulence of MRSA)

The composition provided by the present invention for reducing the virulence of MRSA contains: at least one bacteriophage selected from the group consisting of a bacteriophage identified by accession number NITE BP-693 and a bacteriophage identified by accession number NITE BP-694.

The composition of the present invention is for reducing the virulence of MRSA via the bacteriophage according to the present invention (as described above).

Herein, "reducing the virulence of MRSA" means reducing the characteristics and ability of MRSA to infect another organism to cause an infection in the host. For the reduction of the virulence, for example, if the expression level of a gene associated with the virulence of MRSA (e.g., EsaA, EsaB, EsaC, EssA, EssB, EssC, EsxA, EsxB, RNAIII, LukGH subunit H, LukGH subunit G, thermonuclease, Staphylococcus superantigen-like protein 11 (SSL11), Staphylokinase, staphylococcal enterotoxin type O, each described later in Examples) in MRSA with application of the bacteriophage according to the present invention is lower than that in MRSA without application of the bacteriophage, the determination that "the virulence of MRSA has been reduced" can be made.

For example, the virulence may be reduced by allowing MRSA to acquire resistance to the bacteriophage. Acquisition of resistance to the bacteriophage can be confirmed with the method as described above.

For example, the composition of the present invention is administered to, for example, mammals such as rodents including mice, rats, hamsters, and guinea pigs, primates including humans, chimpanzees, and rhesus monkeys, livestock including pigs, bovines, goats, horses, sheep, and avians, and pet animals including dogs and cats. Humans are preferred subjects.

For example, the composition of the present invention can be formulated into a dosage form such as a tablet, a granule, a powder, a capsule, a syrup, and an injection by means of a conventional method, and a proper drug delivery system (DDS) may be used. In addition, diluents, binders, lubricants, coloring agents, disintegrators, thickeners, preservatives, stabilizers, pH adjusters, and others that are used in common pharmaceuticals can be added. The dosage of the composition can be appropriately set according to the age, body weight, indication, and others of a subject individual.

(4. Brief summary)

As explained hereinbefore, the present invention can provide a composition that contains the bacteriophage according to the present invention and increases the drug sensitivity of MRSA to an antibacterial drug. Use of the composition of the present invention for MRSA, for which previous drugs have failed in exerting effects of disinfection, decolonization, inhibition of growth, and the like, increases the drug sensitivity to an antibacterial drug, as a result allowing the antibacterial drug to exert its effects of disinfection, decolonization, inhibition of growth, and the like for MRSA.

The composition provided by the present invention for treating or preventing an MRSA infection can elicit the effect of an antibacterial drug to treat or prevent an MRSA infection by increasing the drug sensitivity of MRSA to the antibacterial drug via the bacteriophage according to the present invention. Even for MRSA infections for which previous drugs have been ineffective, an effective therapeutic or preventive effect can be elicited by increasing the drug sensitivity to an antibacterial drug via the bacteriophage according to the present invention.

The composition provided by the present invention for reducing the virulence of MRSA can reduce the virulence of MRSA itself via the bacteriophage according to the present invention, and eventually a therapeutic or preventive effect for MRSA infections can be expected.

It should be noted that the present invention also encompasses a method for increasing the drug sensitivity of MRSA to an antibacterial drug, a method for treating or preventing an MRSA infection, and a method for reducing the virulence of MRSA, and each of these methods may include a step of allowing MRSA to acquire resistance to the bacteriophage according to the present invention.

Examples

Hereinafter, the present invention will be specifically described with reference to Examples. However, the present invention is not limited to those Examples.

The present inventors have previously succeeded in isolating bacteriophages having a characteristic to cause bacteriolysis for S. aureus, which are a bacteriophage identified by accession number NITE BP-693 and that identified by accession number NITE BP-694 (φSA012 and φSA039).

In this Example, ability to increase the drug sensitivity of MRSA to an antibacterial drug and differential expressions of genes associated with the toxicity MRSA were evaluated for φSA012 in a test shown in the following.

(Example 1)

Bacteria having acquired phage resistance are known to undergo alteration (loss) of their original function due to the resistance acquirement involving mutations of their genes (trade-off). Accordingly, gene mutations in phage-resistant (φSA012-resistant) MRSA were investigated.

ΦSA012-resistant MRSA (φSA012mts) was obtained as follows. Into 4 mL of LB liquid medium, 40 μL of MRSA2007-13 strain (GCA_018409145.1) bacterial solution and 40 μL of φSA012 ($10^9$ PFU/mL) were mixed, and the resultant was subjected to shaking culture at 37°C for 6 days. Thereafter, bacterial cells were collected by means of centrifugation, washed with 1 mL of PBS, and streaked on LB agar medium. After culturing at 37°C overnight, single colonies formed on the medium were collected, added to 3 mL of LB-Broth, and subjected to shaking culture at 37°C overnight for cloning. The cloned bacterial strains were examined by spot test with soft agar LB medium and temporal turbidimetry with a plate reader, and bacterial strains for which the bacteriolytic activity of φSA012 was not found were used as mutated strains.

The following shows a mutation analysis method for the mutants. The mutant SAm1-201 was subjected to genome extraction, and genomic analysis was performed by using a next-generation sequencer. SAm1-201 was precultured in LB-Broth, and subjected to genome extraction by using a GenElute (R) Bacterial Genomic DNA Kit (Sigma-Aldrich Co. LLC) (performed in accordance with the product instruction). Genomic analysis was outsourced to Bioengineering Lab. Co., Ltd., and sequencing analysis was performed by using the next-generation sequencer DNBSEQ-G400 at 2 × 200 bp. Adapter sequences and low-quality reads were removed by using Trimmomatic (ver. 0.39), and mapping on reference sequences and mutation detection were then performed by using CLC Genomics Workbench 20. As a result, mutations of mgrA and femA were detected as single nucleotide polymorphism, and hence colony PCR was performed for mutations of the two genes with the number of mutants to be investigated further increased, and after that the nucleotide sequences of mgrA and femA of multiple mutants were determined by Sanger sequencing. Bacterial solutions of the mutants were each streaked on LB agar medium and cultured at 37°C overnight, and single colonies were then collected and PCR was performed. The primers used for the PCR reaction are shown in the following.

- femA (Forward): CAAAGCCATCATTCTCACGGG (SEQ ID NO: 1)
- femA (Reverse): CAGAGGGGAAATAGAAAAACTGC (SEQ ID NO: 2)
- mgrA (Forward): GCTCAAAGACAAGTTAATCGCT (SEQ ID NO: 3)
- mgrA (Reverse): ATCAAATGCATGAATGACTTTACCT (SEQ ID NO: 4)

The PCR products obtained were purified by using a FastGene Gel/PCR Extraction Kit (NIPPON Genetics Co., Ltd.) (performed in accordance with the product instruction). Sanger sequencing was outsourced to Hokkaido System Science Co., Ltd.

Information on the mutants obtained is shown below. A mutation of mgrA was found for all the mutants (22/22: 100%), and approximately 70% of the mutants were found to further have a mutation of femA (16/22: 73%) (Table 1). mgrA is known to regulate approximately as many as 350 gene expressions. femA is for an enzyme that synthesizes peptidoglycan, which constitutes external walls of bacteria, and a mutation thereof is expected to largely alter the structure of peptidoglycan. Among three-digit numbers following a mutant name in Table 1, numbers from 100 to 199 indicate having a mutation only in mgrA; and numbers from 200 to 299 indicate having a mutation simultaneously in both of mgrA and femA.

[Table 1]

| strain | | femA | | mgrA | |
|---|---|---|---|---|---|
| | | NT_POS | Type | NT_POS | Type |
| | SAm1-101 | | | | deletion |
| | SAm1-102 | | | | deletion |
| | SAm1-103 | | Non-mutantation | | deletion |
| | SAm1-104 | | | 289/444 | transposon insertion (IS256) |
| | SAm1-105 | | | | deletion |
| | SAm1-106 | | | | deletion |
| | SAm1-201 | 947/1263 | snv (G→T) | 331/444 | insertion (G→GT) |
| | SAm1-202 | 1222/1263 | insertion (G→GCA) | 125/444 | transposon insertion (IS256) |
| | SAm1-203 | 947/1263 | snv (G→T) | 289/444 | transposon insertion (IS256) |
| | SAm1-204 | 1222/1263 | insertion (G→GCA) | 125/444 | transposon insertion (IS256) |
| MRSA2007-13 | SAm1-205 | 1123/1263 | deletion (GAAGATGCTG→G) | 289/444 | transposon insertion (IS256) |
| | SAm1-206 | 1206/1263 | deletion (TAAACCTGTTTACGCAG→T) | 289/444 | transposon insertion (IS256) |
| | SAm1-207 | 1222/1263 | insertion (G→GC) | 373/444 | deletion (GATGAAG→G) |
| | SAm1-208 | 986/1263 | snv (C→A) | 241/444 | insertion (T→TAGACT) |
| | SAm1-209 | 1206/1263 | deletion (TAAACCTGTTTACGCAG→T) | 289/444 | transposon insertion (IS256) |
| | SAm1-210 | 1206/1263 | deletion (TAAACCTGTTTACGCAG→T) | 289/444 | transposon insertion (IS256) |
| | SAm1-211 | 1222/1263 | insertion (G→GCA) | 125/444 | transposon insertion (IS256) |
| | SAm1-212 | 1206/1263 | deletion (TAAACCTGTTTACGCAG→T) | 289/444 | transposon insertion (IS256) |
| | SAm1-213 | 947/1263 | snv (G→T) | 289/444 | transposon insertion (IS256) |
| | SAm1-214 | 1221 | deletion (AG→A) | 143/444 | snv (G→A) |
| | SAm1-215 | 1206/1263 | deletion (TAAACCTGTTTACGCAG→T) | 289/444 | transposon insertion (IS256) |
| | SAm1-216 | 1222/1263 | insertion (G→GCA) | 125/444 | transposon insertion (IS256) |

Subsequently, the infectivity of a phage to φSA012-resistant MRSA obtained in the above (mutants: SAm1-201, SAm1-106) was checked.

The test method is shown in the following. To 3 mL of LB-Top Agar, 100 μL of a precultured test bacterial solution (SAm1-201, SAm1-106) was added, and the resultant was seeded on LB-Agar. Thereon, serially diluted phage solutions (φSA012) ranging from $1 \times 10^2$ pfu/mL to $1 \times 10^8$ pfu/mL and SM-Buffer (negative control) each in 4 μL were added dropwise, and the resultant was cultured at 37°C overnight. The following day, plaques formed by the bacteriolytic activity of the phage in the mutated strains (SAm1-201, SAm1-106) and those in a reference bacterial strain (wild-type strain: MRSA2007-13 strain) were counted, and the EOPs (efficacy of plating: plaque-forming rate) were calculated based on the following calculation formula.

$$\text{EOP} = \text{phage titer for test bacterial strain} / \text{phage titer for reference bacterial strain}$$

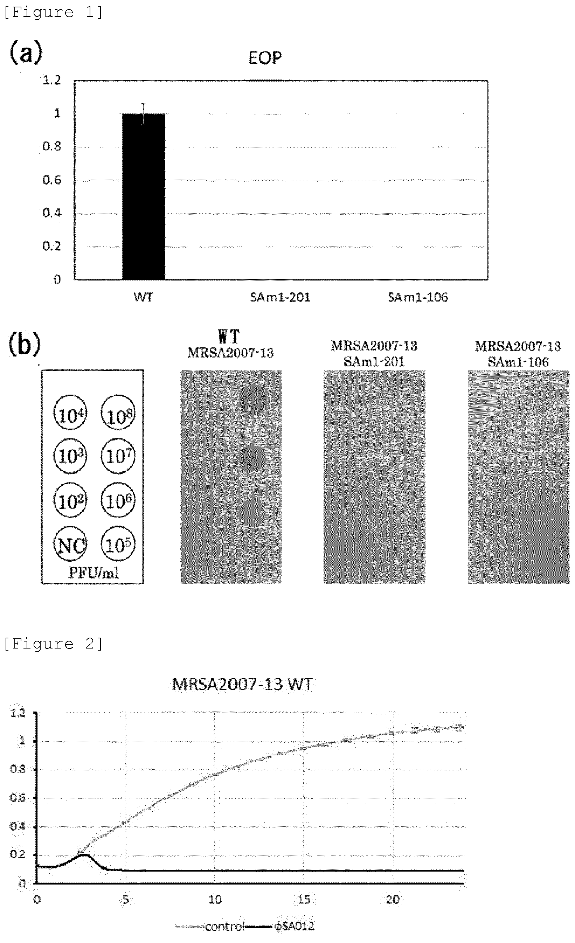

Figure 1 shows the results. For the wild-type strain ("wt" in Figure 1), plaques were formed by addition of φSA012, confirming the infection with φSA012. For the mutated strains (SAm1-201, SAm1-106), by contrast, the plaque-forming ability of φSA012 was not observed, which demonstrated that φSA012 does not infect them (Figure 1(a)). Figure 1(b) shows photographs of states of bacteriolysis for the wild-type strain ("wt" in Figure 1(b)) and the mutated strains (SAm1-201, SAm1-106). Bacteriolysis was not found at all for SAm1-201, and plaques due to bacteriolysis at a level called turbid were observed at $10^8$ pfu/mL (or $10^7$ pfu/mL) for SAm1-106, though no clear plaque was found (Figure 1(b)). Thus, bacteriolysis by φSA012 was not found for any of the mutated strains, and it was confirmed that, with certainty, the mutated strains are not infected by φSA012.

Subsequently, the growth-inhibitory effect of a phage (φSA012) for φSA012-resistant MRSA obtained in the above (mutants: SAm1-201, SAm1-106) was examined.

The test method is shown in the following. This test was performed by means of turbidimetry. Turbidimetry enables real-time measurement of the relationship between the infectivity of a phage and bacterial growth. The specific way of turbidimetry will be explained. The growth activities of a wild-type strain (MRSA2007-13 strain) and mutants (SAm1-201, SAm1-106) in the absence of φSA012 (control) or in the presence of φSA012 were each measured with a temporal turbidimetry method using a 96-well plate. A bacterial solution (wild-type strain (MRSA2007-13 strain) or a mutant (SAm1-201, SAm1-106)) precultured overnight was diluted with LB-Broth to reach $OD_{600} \approx 1.0$, and further 10-fold diluted. To each well, 90 μL of the bacterial solution and 10 μL of φSA012 ($1 \times 10^8$ pfu/mL) were added. The plate was set in a Sunrise Rainbow Thermo RC (Tecan Japan Co., Ltd.), and shaking culture was performed at 37°C for 24 hours while absorbance values at a wavelength of 590 nm were measured every 15 minutes, and the growth activity was evaluated.

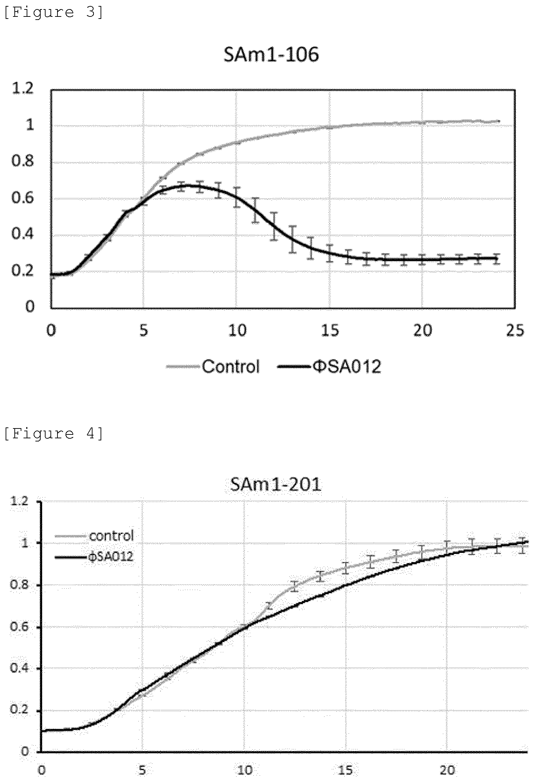

Figures 2 to 4 show the results. In each of Figures 2 to 4, the ordinate represents turbidity, the abscissa represents time (h), and a gray graphic line and a black graphic line represent bacterial growth without the phage and that with the phage, respectively. The wild-type strain (MRSA2007-13 strain) immediately underwent bacteriolysis by φSA012, lacking increased turbidity, which is indicative of bacterial growth; thus, it was confirmed that bacterial growth was inhibited by the phage (Figure 2). For the mutant with one mutation in mgrA (SAm1-106), on the other hand, the degree of inhibition of bacterial growth by φSA012 was weakened, and increased turbidity was found in the early stage (Figure 3). Moreover, the mutant having a mutation in both mgrA and femA (SAm1-201) exhibited growth similar to that of the wild-type strain without bacterial growth inhibition by the phage, and thus the bacterium was confirmed to have acquired resistance to the phage (Figure 4). Thus, it was confirmed that, with certainty, the bacterial growth-inhibitory effect of the phage (φSA012) is not elicited for the mutants, and, in particular, it was revealed that the mutated strain having a mutation in both mgrA and femA had a higher acquired phage resistance of MRSA.

(Example 2)

MRSA having acquired phage resistance was expected to have altered drug sensitivity by trade-off. Accordingly, examination was performed on alteration of drug sensitivity accompanying acquirement of phage resistance.

The drug sensitivities of a wild-type strain (MRSA2007-13 strain) and a mutant (SAm1-201) to β-lactam antibacterial drugs were measured in a drug sensitivity test (disk method). The test method will be explained. The wild-type strain (MRSA2007-13 strain) or the mutant (SAm1-201) was streaked on LB-Agar, and cultured at 37°C overnight. The following day, single colonies were collected, added to 5 mL of Trypticase soy broth, and subjected to shaking culture at 37°C overnight. After culturing, the bacterial solution was adjusted to McFarland turbidity No. 0.5, and evenly smeared on Mueller-Hinton II agar medium with a sterile cotton swab. After disposing sensitivity disks on the agar medium, culture was performed at 37°C for 24 hours, and the diameter of an inhibitory zone formed around each disk was measured in millimeters.

In Table 2, codes for β-lactam antibacterial drugs are as follows.

| | |
|---|---|
| MPIPC: | oxacillin |
| AMPC: | amoxicillin |
| CEX: | cephalexin |
| CPDX: | cefpodoxime |
| FRPM: | faropenem |
| IPM: | imipenem |
| MEPM: | meropenem |

Table 2 shows the results. The wild-type strain ("WT" in Table 2) exhibited resistance (R) to all the β-lactam antibacterial drugs. On the other hand, the mutant (SAm1-201) was demonstrated to have sensitivity (S) to the β-lactam antibacterial drugs.

[Table 2]

| Antibacterial drug | | MRSA2007-13 | |
|---|---|---|---|
| | | WT | SAm1-201 |
| β –Lactam antibacterial drug | MPIPC | R | S(35) |
| | AMPC | R | S(34) |
| | CEX | R | S(29) |
| | CPDX | R | S(28) |
| | FRPM | R | S(50≦) |
| | IPM | R(9) | S(44) |
| | MEPM | R(7) | S(28) |

Next, the drug sensitivities of a wild-type strain (MRSA2007-13 strain) and mutated strains having a mutation in both mgrA and femA (SAm1-201, SAm1-206, SAm1-210) to antibacterial drugs were measured in a drug sensitivity test (MIC method). The test method will be explained. A bacterial solution (a wild-type strain or a mutant) precultured overnight was diluted with sterile physiological saline to reach $OD_{600} \approx 0.26$, and 25 μL of the bacterial solution was added to 12 mL of Mueller-Hinton liquid medium (Eiken Chemical Co., Ltd.). To each well of a dry plate (Eiken Chemical Co., Ltd.), 100 μL of the bacterial solution was added, and cultured for 20 hours. After culturing, the MIC was determined based on determination criteria for the dry plate.

In Table 3, codes for antibacterial drugs are as follows.

AMPC:      amoxicillin
CEZ:        cefazolin
MPIPC:     oxacillin
PCG:        penicillin
S/A:         sulbactam/ampicillin
CDTR:       cefditoren
CFPN:       cefcapene
FRPM:       faropenem
MEPM:      meropenem
IPM:         imipenem
OTC:        oxytetracycline
DOXY:       doxycycline
ERFX:       enrofloxacin

Table 3 shows the results. In the MIC method, less requirement of a drug (lower MIC value) is rated as higher drug sensitivity. The mutants having both a mutation of mgrA and a mutation of femA exhibited 4 to 1000 times or higher drug sensitivity to antibacterial drugs than the wild-type strain ("WT" in Table 3) exhibited. In particular, as demonstrated by the data "WT: >128" vs. "SAm1-201: 0.125" for CEZ (cefazolin), the drug sensitivity of the mutated strain to the antibacterial drug was found to be dramatically higher than that of the wild-type strain. While there is a previous report that femA mutation in methicillin-sensitive Staphylococcus aureus increases the sensitivity thereof to oxacillin (Proc Natl Acad Sci U S A. 2021 Mar 9; 118 (10): e2008007118. doi: 10.1073/pnas.2008007118.), the present results give the first report that the sensitivity of MRSA to antibacterial drugs is significantly enhanced.

[Table 3]

| Antibacterial drug | | WT | MRSA2007-13 (having mgrA mutation and FemA mutation) | | |
|---|---|---|---|---|---|
| | | | SAm1-201 | SAm1-206 | SAm1-210 |
| β –Lactam antibacterial drug | AMPC | 64 | 8 | 16 | 2 |
| | CEZ | >128(R) | 0.125(S) | 1 | 0.25 |
| | MPIPC | >128 | 0.125 | 4 | 0.125 |
| | PCG | >16 | 2 | 2 | 4 |
| | S/A | 8/16(I) | 0.5/1(S) | 0.5/1(S) | 0.5/1(S) |
| | CDTR | >2 | <0.5 | <0.5 | <0.5 |
| | CFPN | >2 | <0.5 | <0.5 | <0.5 |
| | FRPM | >128 | 0.125> | 0.25 | 0.125 |
| | MEPM | 32 | 0.125> | 4 | 0.125 |
| | IPM | 64 | 0.125> | 1 | 0.125 |
| | OTC | 128 | 64 | 128 | 128 |
| | DOXY | 8 | 4 | 8 | 4 |
| | ERFX | 128 | 64 | 64 | 32 |

Thus, it was demonstrated that the drug sensitivity of MRSA to antibacterial drugs including β-lactam ones is increased by allowing MRSA to acquire phage resistance with a bacteriophage (φSA012).

(Example 3)

Now that it has been revealed that acquirement of phage resistance causes alteration of sensitivity to antibacterial drugs; next, a phage and an antibacterial drug (oxacillin) were both added to a wild-type strain of MRSA from the beginning of test to examine the growth-inhibitory effect.

The test method is shown in the following. This test was performed by means of turbidimetry. A bacterial solution (wild-type strain (MRSA2007-13 strain)) precultured overnight was diluted with LB-Broth to reach $OD_{600} \approx 1.0$, and further 10-fold diluted ($\approx 1.0 \times 10^8$ cfu/mL). Groups were established as follows.

- Control: bacterium 90 μL + SM buffer 10 μL
- phi: bacterim 90 μL + φSA012 10 μL (final concentration :t $1.0 \times 10^7$ pfu/m (MOI :t 0.1))
- Abx: bacterium 99 μL + oxacillin sodium 1 mL (final concentration: 16 μg/mL)
- phi+Abx: bacterium 89 μL + phage 10 μL (final concentration $\approx 1.0 \times 10^7$ pfu/m (MOI $\approx$ 0.1)) + oxacillin sodium 1 μL (final concentration: 16 μg/mL)

The plate was set in a Sunrise Rainbow Thermo RC (Tecan Japan Co., Ltd.), and shaking culture was performed at 37°C for 90 hours while absorbance values at a wavelength of 590 nm were measured every 15 minutes.

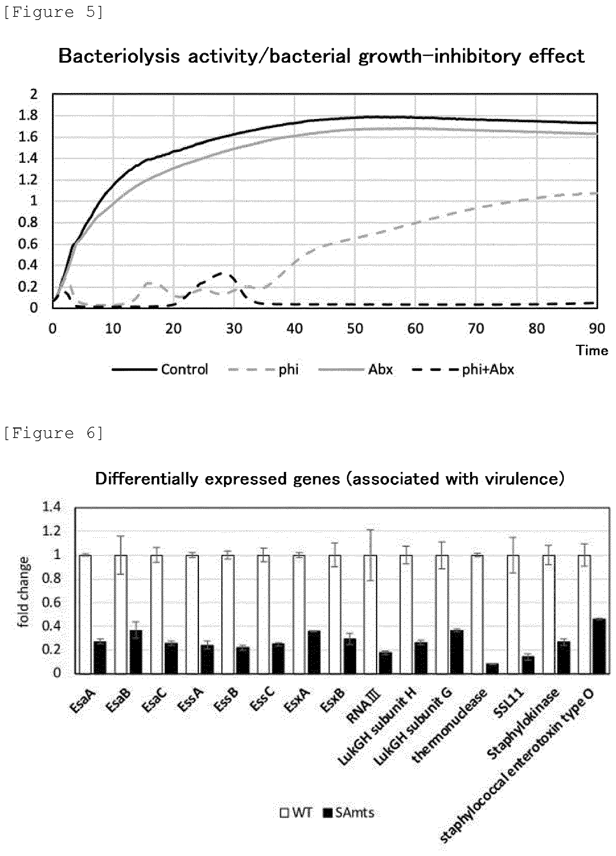

Figure 5 shows the results. The antibacterial drug alone (Abx: gray solid line) failed in inhibiting bacterial growth. The phage (φSA012) alone (phi: gray dashed line) succeeded in inhibiting bacterial growth to some extent, but the regrowth of the bacterium was found after 40 hours from the beginning of culture. For the group with addition of the phage (φSA012) and oxacillin (phi+Abx: black dashed line), bacterial growth had been successfully inhibited for 90 hours after the beginning of culture, and the regrowth of the bacterium was not observed during the 90 hours.

These results demonstrated that a cocktail formed of a phage (φSA012) and an antibacterial drug increases the sensitivity of MRSA to the antibacterial drug, successfully ensuring inhibition of the growth of MRSA.

(Example 4)

With considering the result that the phage-resistant (ΦSA012-resistant) MRSA strain contains 100% mutation in mgrA, differential gene expression due to mgrA mutation was examined through RNA-seq analysis.

Methods of RNA extraction and RNA-seq analysis are shown in the following. A wild-type strain (MRSA2007-13 strain) and a phage-induced mutant (SAm1-201) were cultured in LB-Broth until OD$_{600}$ ≈ 1.0 was attained. After 1 mL of each bacterial solution was centrifuged to form a pellet, the pellet was suspended in 1 mL of TRIzol Reagent (Thermo Fisher Scientific), and 500 μL of Zirconia beads was added thereto and bacterial cells were broken by means of a pulverizer. After pulverization, centrifugation was performed to collect the supernatants, and RNA fractions were extracted with chloroform and ethanol. Library preparation and RNA-seq analysis were outsourced to Rhelixa, Inc. Libraries were produced with a Ribo-Zero Plus rRNA Depletion Kit and a NEBNext Ultra Directional RNA Library Prep Kit for Illumina, and sequencing analysis was performed by using an Illumina NovaSeq 6000 at 150 bp × 2. Adapter sequences and low-quality reads were removed by using Trimmomatic (ver. 0.39), and mapping on reference sequences was performed by using HISAT2 (ver. 2.1.0). Reads were counted for every gene by using featureCounts (ver. 2.0.1), TMM normalization was then performed by using TCC-GUI, and differentially expressed genes were detected.

A previous report says that mgrA serves as a transcriptional regulator to regulate about 350 genes including genes involved in virulence and genes involved in drug efflux pumps. Accordingly, examination was performed on differential gene expression due to mgrA mutation resulting from acquirement of phage resistance.

Figure 6 shows the results. It was shown that the expression levels of 15 genes involved in virulence in the ϕSA012-resistant MRSA (SAm1-201) were as low as about 1/2 to 1/10 of those in the wild-type strain (MRSA2007-13 strain). Those genes will be described below. The present results suggested that the MRSA having acquired phage resistance has reduced virulence. In other words, it was demonstrated that the virulence of MRSA is reduced by allowing MRSA to acquire phage resistance with a bacteriophage (ϕSA012).

<EsaA, EsaB, EsaC, EssA, EssB, EssC, EsxA, EsxB>

T7SS is composed of four membrane proteins (EsaA, EssA, EssB, EssC), two intracellular proteins (EsaB, EsaG), five secreted substrates (EsxA, EsxB, EsxC, EsxD, EsaD), and EsaE, which interacts with the secreted substrates. T7SS plays an important role in ensuring the virulence of Staphylococcus aureus. It has been reported that deletion of all or specific factors (EsxA, EssB, EssC, EsxC, EsxB, EsaB, EsaD, EsaE) of T7SS results in reduction in virulence in a mouse infection model.

<RNAIII>

A mechanism in which a bacterium senses the population density of the same species and controls the productions of substances in response to the population density is called quorum sensing. Some of the virulence factors of Staphylococcus aureus are regulated through quorum sensing called the accessory gene regulator (Agr) regulatory system. Activation of the Agr system through quorum sensing causes transcription of RNAIII, and this RNAIII directly or indirectly regulates transcription and expression of virulence factors.

<LukGH subunit H, LukGH subunit G>

LukGH, also called LukAB, is one of leukocidins. Leukocidins are toxin components that form holes in leukocytes such as neutrophils to destroy them.

<Thermonuclease>

Thermonuclease (nuc) is extracellularly secreted, and capable of degrading DNA present out of cells. In particular, neutrophils are known to destruct themselves against Staphylococcus aureus and trap the bacterial cells by discharging their own DNA (NETs: neutrophil extracellular traps). Thermonuclease acts on NETs to assist in escaping form the trap.

<Staphylococcus superantigen-like protein 11 (SSL11)>

SSL is a protein the structure of which is similar to that of a superantigen of Staphylococcus aureus. SSL11 suppresses immunity by preventing neutrophils from being activated and rolling onto vascular vessels. Moreover, SSL11 is known to inhibit cell adhesion and motility.

<Staphylokinase>

Staphylokinase (sak) is known to bind to plasminogen and $\alpha$-defencin, which is secreted from neutrophils. On being bound to sak, plasminogen becomes plasmin to activate. Plasmin functions as a broad-spectrum proteinase, and contributes to the invasion of Staphylococcus aureus into surrounding tissue. The antibacterial peptide $\alpha$-defencin undergoes the deactivation of the action on binding to sak, allowing Staphylococcus aureus to survive. Gene expression for sak is known to be positively regulated by the Agr system as quorum sensing.

<Staphylococcal enterotoxin type O>

SEO is one of enterotoxins secreted by Staphylococcus aureus, and known to cause vomiting on being inoculated. In addition, SEO is known to induce secretion of IL-1$\beta$ from neutrophils via the inflammasome pathway.

Differential expressions of NorB and Tet, which are genes associated with drug efflux pumps, were examined through the same RNA-seq analysis as above. NorB and Tet are genes involved in drug efflux pumps, and causal genes for multiple-drug resistance. Reduction in expressions of those genes inhibits the effluxes of antibacterial drugs in bacteria, thus increasing the sensitivity to antibacterial drugs.

Figure 7 shows the results. It was revealed that the expression levels of NorB and Tet, which are involved in drug efflux pumps, in the $\phi$SA012-resistant MRSA (SAm1-201) were as low as 1/5 or less of those in the wild-type strain (MRSA2007-13WT). Accordingly, reduction in expressions of genes involved in drug efflux pumps was expected to be one of mechanisms of action for increasing the drug sensitivity of MRSA to antibacterial drugs with a phage ($\phi$SA012). Particularly suggested was the possibility that reduction in expression of NorB increases the sensitivity to new quinolone antibacterial drugs (such as ERFX), and reduction in expression of Tet increases the sensitivity to tetracycline antibacterial drugs (such as OTC). In short, it was demonstrated that the drug sensitivity of MRSA to antibacterial drugs is increased by allowing MRSA to acquire phage resistance with a bacteriophage ($\phi$SA012).

(Example 5)

The results of RNA-seq analysis for the MRSA2007-13 SAm1-201 strain, which is a phage-resistant bacterium having mgrA mutation, found reduced expression levels of various virulence genes (Example 4), and hence the virulence of the phage-resistant bacterium was evaluated by using MRSA infection model mice.

(Evaluation of virulence with mouse peritonitis model)

Bacterial solutions of a wild-type strain (MRSA2007-13 strain) and a phage-induced mutant (phage-resistant bacterium MRSA2007-13 SAm1-201) precultured overnight were each added to fresh LB broth, and subjected to shaking culture at 37°C until $OD_{600} \approx 1.0$ was attained. The bacterial solutions were centrifuged (8,000 $\times$g, 4°C, 5 minutes), and the pellets were each suspended in PBS to reach $1.0 \times 10^{10}$ CFU/mL. To 8-week-old female BALB/c mice (Sankyo Labo Service Corporation, INC., Tokyo, Japan), the bacterial solutions each in 100 $\mu$L were intraperitoneally administered ($1.0 \times 10^9$ CFU/head), and their survival was checked every 12 hours for 48 hours after administration. The present animal experiment was carried out under the approval of the Rakuno Gakuen University Animal Experiment Committee (approval number: VH21A13). Groups each consisting of eight mice (n = 8) were tested with log-rank test, in which p < 0.05 was considered as a significant difference.

(Evaluation of virulence with mouse skin abscess model)

Bacterial solutions of a wild-type strain (MRSA2007-13 strain) and a phage-induced mutant (phage-resistant bacterium MRSA2007-13 SAm1-201) precultured overnight were each added to fresh LB broth, and subjected to shaking culture at 37°C until $OD_{600} \approx 1.0$ was attained. The bacterial solutions were centrifuged (8,000 $\times$g, 4°C, 5 minutes), and the pellets were each suspended in PBS to reach $2.0 \times 10^9$ CFU/mL. To 8-week-old female BALB/c mice (Sankyo Labo Service Corporation, INC., Tokyo, Japan), a three-anesthetic mixture (0.75 mg/kg medetomidine, 4 mg/kg midazolam, 5 mg/kg butorphanol) was intraperitoneally administered, the part ranging from the flank to the femur in each side was shaved with hair clippers under anesthesia, and the surfaces of the skin were disinfected with 70% alcohol. Thereafter, the bacterial solutions each in 50 $\mu$L were subcutaneously administered at a site around from the flank to the femur in each side ($1.0 \times 10^8$ CFU/site), and the mice were then allowed to wake up with 0.75 mg/kg atipamezole. After administration, the abscess sizes were measured (major axis (mm) $\times$ minor axis (mm) = mm$^2$) once per day. On day 7 after administration, the mice were euthanized with anesthetic treatment, and the abscess sizes were measured and the lesion sites were photographed. To determine the bacterial counts in the lesion sites, each lesion site was collected and finely cut into pieces, to which 200 $\mu$L of PBS and $\phi$5 mm zirconia balls were added, the tissue was pulverized to produce a lysate, and the bacterial count was determined by means of plate dilution. The present animal experiment was carried out under the approval of the Rakuno Gakuen University Animal Experiment Committee (approval number: VH21A13).

Figure 8(a) shows the results of the evaluation of virulence with the mouse peritonitis model. The mice with intraperitoneal administration of the wild-type strain (MRSA2007-13 strain) were all died within 24 hours (Figure 8(a): black line). On the other hand, the 48-hour survival rate of the mice with administration of the phage-induced mutant (SAm1-201) was 50% (Figure 8(a): gray line). These results demonstrated that the MRSA having acquired phage resistance had lower virulence, giving significantly higher survival rates to mice.

Figures 8(b) to 8(e) show the results of the evaluation of virulence with the mouse skin abscess model. Of the three mice with administration of the wild-type strain (MRSA2007-13 strain), one died of sepsis-like symptoms 2 days after administration, and another underwent the commissure of the left and right lesion sites throughout the observation period; thus, measurement of abscess size was unavailable for the two. While abscess was found over a broad range in the mice with administration of the wild-type strain (MRSA2007-13 strain) (Figure 8(b)), by contrast, the range of abscess was limited and the abscess sizes were significantly small in the mice with administration of the phage-induced mutant (SAm1-201) (Figure 8(c)). The transitions of abscess size over 7 days after administration demonstrated that the mice with administration of the phage-induced mutant (SAm1-201) (Figure 8(d): dashed line) exhibited significantly smaller abscess sizes than the mice with administration of the wild-type strain (MRSA2007-13 strain) (Figure 8(d): solid line) exhibited (Figure 8(d)). The tissues at the lesion sites 7 days after administration were excised, and the bacterial counts in the tissues were determined to find that the bacterial count in a mouse with administration of the phage-induced mutant (SAm1-201) was significantly smaller than that in a mouse with administration of the wild-type strain (MRSA2007-13 strain), the former bacterial count detected being about 1/50 of the latter (Figure 8(e)).

Thus, in both the mouse peritonitis model and the mouse skin abscess model, the phage-induced mutant (SAm1-201) was revealed to have lower virulence to mice than the wild-type strain (MRSA2007-13 strain). In short, it was demonstrated that the virulence of MRSA is reduced by allowing MRSA to acquire phage resistance with a bacteriophage ($\phi$SA012).

The present invention permits various embodiments and modifications, without departing from the spirit and scope of the present invention in a broad sense. The embodiments described above are for describing the present invention, being not for limiting the scope of the present invention. Specifically, the scope of the present invention is shown not by embodiments but by claims. Furthermore, various modifications applied within the scope of claims and the scope of a meaning of the invention equivalent thereto are regarded to fall within the scope of the present invention.

The present invention is based on Japanese Patent Application No. 2021-137252 filed on August 25, 2021. The specification, claims, and drawings of Japanese Patent Application No. 2021-137252 are totally incorporated herein by reference.

Accession Numbers

**[0103]**

(1) Statement of identity: 003+$\phi$SA012
(2) Accession number: NITE BP-693
(3) Date of accession: December 25, 2008
(4) Depositary: National Institute of Technology and Evaluation

(1) Statement of identity: 0031+$\phi$SA039
(2) Accession number : NITE BP-694
(3) Date of accession: December 25, 2008
(4) Depositary: National Institute of Technology and Evaluation

**Claims**

1. A composition for increasing drug sensitivity of methicillin-resistant Staphylococcus aureus (MRSA) to an antibacterial drug, the composition comprising: at least one bacteriophage selected from the group consisting of a bacteriophage identified by accession number NITE BP-693 and a bacteriophage identified by accession number NITE BP-694.

2. The composition according to claim 1, wherein the drug sensitivity is increased by allowing MRSA to acquire resistance to the bacteriophage.

3. The composition according to claim 1 or 2, wherein the antibacterial drug is a β-lactam antibacterial drug.

4. A composition for treating or preventing an MRSA infection, the composition comprising: at least one bacteriophage selected from the group consisting of a bacteriophage identified by accession number NITE BP-693 and a bacteriophage identified by accession number NITE BP-694; and an antibacterial drug.

5. The composition according to claim 4, wherein MRSA is allowed to acquire resistance to the bacteriophage.

6. The composition according to claim 4 or 5, wherein the antibacterial drug is a β-lactam antibacterial drug.

7. A composition for reducing virulence of MRSA, the composition comprising: at least one bacteriophage selected from the group consisting of a bacteriophage identified by accession number NITE BP-693 and a bacteriophage identified by accession number NITE BP-694.

8. The composition according to claim 7, wherein the virulence is reduced by allowing MRSA to acquire resistance to the bacteriophage.

[Figure 1]

(a) EOP

(b)

[Figure 2]

MRSA2007-13 WT

[Figure 3]

SAm1-106

[Figure 4]

SAm1-201

[Figure 5]

## Bacteriolysis activity/bacterial growth-inhibitory effect

[Figure 6]

## Differentially expressed genes (associated with virulence)

[Figure 7]

**Differentially expressed genes (associated with drug efflux pumps)**

[Figure 8]

(a)

$1 \times 10^9$ cfu/head (i.p.)

MRSA2007-13
MRSA2007-13 SAm1-201

$p < 0.05$

(b)

(c)

(d)

Skin lesion area

MRSA2007-13     MRSA2007-13 SAm1-201

(e)

$1 \times 10^8$ cfu/site (s.c.)

### INTERNATIONAL SEARCH REPORT

| International application No. |
| --- |
| **PCT/JP2022/031783** |

| A. | CLASSIFICATION OF SUBJECT MATTER |
| --- | --- |

*A61K 35/76*(2015.01)i; *A61K 31/43*(2006.01)i; *A61K 31/545*(2006.01)i; *A61K 45/00*(2006.01)i; *A61P 31/04*(2006.01)i; *A61P 43/00*(2006.01)i

FI:　A61K35/76 ZNA; A61K31/43; A61K31/545; A61K45/00; A61P31/04; A61P43/00 121

According to International Patent Classification (IPC) or to both national classification and IPC

| B. | FIELDS SEARCHED |
| --- | --- |

Minimum documentation searched (classification system followed by classification symbols)

A61K35/76; A61K31/43; A61K31/545; A61K45/00; A61P31/04; A61P43/00

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/MEDLINE/EMBASE/BIOSIS (STN)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| Y | JP 2011-50373 A (WAKO PURE CHEM. IND., LTD.) 17 March 2011 (2011-03-17) claims, examples, particularly, examples 6 | 1-8 |
| Y | KEBRIAEI, R. et al. Bacteriophage-Antibiotic Combination Strategy: an Alternative against Methicillin-Resistant Phenotypes of Staphylococcus aureus. Antimicrobial Agents and Chemotherapy. 2020, vol. 64, no. 7, e00461-20, pp. 1-6 abstract, table 1, fig. 1, table 2 | 1-6 |
| Y | CAPPARELLI, R. et al. Bacteriophage-Resistant Staphylococcus aureus Mutant Confers Broad Immunity against Staphylococcal Infection in Mice. PLoS ONE. 2010, vol. 5, no. 7, e11720, pp. 1-13 abstract, fig. 4 | 7-8 |
| A | ZSCHACH, H. et al. Use of a Regression Model to Study Host-Genomic Determinants of Phage Susceptibility in MRSA. Antibiotics. 2018, vol. 7, 9, pp. 1-16 abstract, p. 7, last paragraph | 1-8 |

☑ Further documents are listed in the continuation of Box C.　　☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **27 October 2022** | **15 November 2022** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

## INTERNATIONAL SEARCH REPORT

| International application No. |
| --- |
| **PCT/JP2022/031783** |

### C.    DOCUMENTS CONSIDERED TO BE RELEVANT

| Category* | Citation of document, with indication, where appropriate, of the relevant passages | Relevant to claim No. |
| --- | --- | --- |
| A | MAIDHOF, H. et al. femA, which encodes a factor essential for expression of methicillin resistance, affects glycine content of peptidoglycan in methicillin-resistant and methicillin-susceptible staphylococcus aureus strains. Journal of Bacteriology. 1991, vol. 173, no. 11, pp. 3507-3513<br>    abstract, p. 3508, right column, last paragraph to p. 3509, first paragraph, fig. 3 | 1-8 |
| A | TROTONDA, M. P. et al. Role of mgrA and sarA in Methicillin-Resistant Staphylococcus aureus Autolysis and Resistance to Cell Wall-Active Antibiotics. The Journal of Infectious Diseases. 2009, vol. 199, no. 2, pp. 209-218<br>    abstract, fig. 1, 2 | 1-8 |
| A | CHEN, L. et al. The Role of graRS in Regulating Virulence and Antimicrobial Resistance in Methicillin-Resistant Staphylococcus aureus. Frontiers in Microbiology. 16 August 2021, vol. 12, 727104, pp. 1-12<br>    fig. 3 | 1-8 |
| P, X | 中村暢宏 ほか, MRSAのファージ耐性獲得によるβラクタム系抗菌薬の再感受性化, 日本細菌学雑誌, 21 February 2022, vol. 77, no. 1, p. 111, (NAKAMURA, Nobuhiro et al. Bacteriophage-resistant variants of MRSA are resensitized to β-lactam antibiotics. Japanese Journal of Bacteriology.)<br>    abstract | 1-8 |
| P, X | 中村暢宏 ほか, 黄色ブドウ球菌におけるファージ耐性化と病原性低下のトレードオフ, 緑膿菌感染症研究会プログラム•抄録集, 10 February 2022 (received date), vol. 56, p. 28, (NAKAMURA, Nobuhiro et al. A trade-off between bacteriophage resistance and virulence reduction in Staphylococcus aureus.), non-official translation (Program • Abstracts of Pseudomonas Aeruginosa Infection Research Group.)<br>    abstract | 1-8 |
| P, X | 西田啓汰 ほか, 黄色ブドウ球菌におけるファージ耐性化機構の解明, 日本獣医学会学術集会講演要旨集, 07 September 2021, vol. 164, pp. 10-16, (Event Date: 07 September 2021 to 13 September 2021), non-official translation (NISHIDA, Keita et al. Elucidation of phage resistance mechanism in Staphylococcus aureus. Proceedings of the Annual Meeting of the Japan Society of Veterinary Science.)<br>    abstract | 1-8 |

Form PCT/ISA/210 (second sheet) (January 2015)

**INTERNATIONAL SEARCH REPORT**

International application No.

**PCT/JP2022/031783**

| Box No. I | Nucleotide and/or amino acid sequence(s) (Continuation of item 1.c of the first sheet) |

1. With regard to any nucleotide and/or amino acid sequence disclosed in the international application, the international search was carried out on the basis of a sequence listing:

   a. ☑ forming part of the international application as filed:

      ☑ in the form of an Annex C/ST.25 text file.

      ☐ on paper or in the form of an image file.

   b. ☐ furnished together with the international application under PCT Rule 13*ter*.1(a) for the purposes of international search only in the form of an Annex C/ST.25 text file.

   c. ☐ furnished subsequent to the international filing date for the purposes of international search only:

      ☐ in the form of an Annex C/ST.25 text file (Rule 13*ter*.1(a)).

      ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

"The form of Annex C/ST.25 text file" above shall read as "the form of ST.26.".

Form PCT/ISA/210 (continuation of first sheet) (January 2015)

| INTERNATIONAL SEARCH REPORT<br>Information on patent family members | | International application No.<br>**PCT/JP2022/031783** |
|---|---|---|

| Patent document<br>cited in search report | Publication date<br>(day/month/year) | Patent family member(s) | Publication date<br>(day/month/year) |
|---|---|---|---|
| JP 2011-50373 A | 17 March 2011 | (Family: none) | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2011050373 A

- JP 2021137252 A

**Non-patent literature cited in the description**

- **BRANDON A BERRYHILL.** *Proc Natl Acad Sci U S A.,* 09 March 2021, vol. 118 (10), e2008007118

- *Proc Natl Acad Sci U S A.,* 09 March 2021, vol. 118 (10), e2008007118